# EUROPEAN PATENT SPECIFICATION

(11) **EP 4 357 886 B1**
(45) Date of publication and mention of the grant of the patent: **02.07.2025**
(21) Application number: 22306601.0
(22) Date of filing: 21.10.2022
(51) Int. Cl.: G06F 3/01, G06N 20/00, G06N 3/08, A61B 5/24, A61B 5/11, A61B 5/00, A61B 5/16

(54) **SYSTEM, METHOD AND COMPUTER PROGRAM FOR DETECTING AND CONFIRMING AN EMOTION OF A USER**
SYSTEM, VERFAHREN UND COMPUTERPROGRAMM ZUR ERKENNUNG UND BESTÄTIGUNG EINER EMOTION EINES BENUTZERS
SYSTÈME, PROCÉDÉ ET PROGRAMME INFORMATIQUE POUR DÉTECTER ET CONFIRMER UNE ÉMOTION D'UN UTILISATEUR

(43) Date of publication of application: 24.04.2024
(73) Proprietor: Essilor International, 94220 Charenton-Le-Pont (FR)
(72) Inventor: LE CAIN, Aurélie, 94000 CRETEIL (FR); BERNARDIN, Delphine, MONTREAL, H2V1C3 (CA); BARANTON, Konogan, 77450 MONTRY (FR); TATUR, Guillaume, 77144 MONTEVRAIN (FR)
(74) Representative: Plasseraud IP

(56) References cited:
- GEORGE RIGAS ET AL: "Towards Driver's State Recognition on Real Driving Conditions", INTERNATIONAL JOURNAL OF VEHICULAR TECHNOLOGY, vol. 2011, 1 January 2011 (2011-01-01), pages 1 - 14, XP055723415, ISSN: 1687-5702, DOI: 10.1155/2011/617210
- LEE BOON GIIN ET AL: "Wearable Mobile-Based Emotional Response-Monitoring System for Drivers", IEEE TRANSACTIONS ON HUMAN-MACHINE SYSTEMS, vol. 47, no. 5, 1 October 2017 (2017-10-01), Piscataway, NJ, USA, pages 636 - 649, XP093029804, ISSN: 2168-2291, Retrieved from the Internet <URL:https://ieeexplore.ieee.org/stampPDF/getPDF.jsp?tp=&arnumber=7852466> DOI: 10.1109/THMS.2017.2658442
- KWON JANGHO ET AL: "Emotion Recognition Using a Glasses-Type Wearable Device via Multi-Channel Facial Responses", IEEE ACCESS, IEEE, USA, vol. 9, 19 October 2021 (2021-10-19), pages 146392 - 146403, XP011886706, DOI: 10.1109/ACCESS.2021.3121543

## Description

### FIELD OF THE INVENTION

The present invention relates to the field of affective computing.

More particularly, the present invention relates to a system for determining an emotion of a user, to a corresponding method and to a corresponding computer program.

### BACKGROUND OF THE INVENTION

Emotion recognition is the process of identifying human emotion. Use of technology to perform emotion recognition is a relatively nascent research area, which is said to contribute in the emergence of the so-called emotional or emotive Internet.

To date, the most work has been conducted on automating the recognition of conversational data, including facial expressions from video, spoken expressions from audio, or written expressions from text. Generally, this recognition works best if it uses multiple modalities in context.

Studies have also shown that the human visual system is highly sensitive to biological motion and, when presented with a biological motion pattern of any individual, is capable of extracting information about emotions, intentions, personality traits, motor style and biological attributes of the individual. Models have then been developed in the recent years for automatically analyzing biological motion patterns and determining associated emotions. These models use, both in training and in production, datasets representing global motion of the body, for instance datasets obtained using full-body motion sensing input devices. Emotions have been successfully detected during subject walking activity through the analysis of the dynamics of full-body landmarks corresponding to body joints in a laboratory environment. Recording these dynamics however requires specific equipment and a stable methodology, which are limiting factors for a widespread use of biological motion pattern analysis.

There is therefore a need for methods and products that allow reliably determining emotions of a user even when no conversational data is available.

Any relevant data should further be obtainable using minimal hardware, preferably using no more than a mobile device provided to the user.

GEORGE RIGAS ET AL: "Towards Driver's State Recognition on Real Driving Conditions", INTERNATIONAL JOURNAL OF VEHICULAR TECHNOLOGY, vol. 2011, 1 January 2011 (2011-01-01), pages 1-14, XP055723415, ISSN: 1687-5702, DOI: 10.1155/2011/617210 discloses a system for detecting drivers' stress and fatigue based on physiological signals, video recordings, and environmental information.

LEE BOON GIIN ET AL: "Wearable Mobile-Based Emotional Response-Monitoring System for Drivers", IEEE TRANSACTIONS ON HUMAN-MACHINE SYSTEMS, vol. 47, no. 5, 1 October 2017 (2017-10-01), pages 636-649, XP093029804, Piscataway, NJ, USA ISSN: 2168-2291, DOI: 10.1109/THMS.2017.2658442 discloses a mobile-based wearable device that monitors a driver's negative emotional responses based on physiological signals and head motions.

KWON JANGHO ET AL: "Emotion Recognition Using a Glasses-Type Wearable Device via Multi-Channel Facial Responses", IEEE ACCESS, IEEE, USA, vol. 9, 19 October 2021 (2021-10-19), pages 146392-146403, XP011886706, DOI: 10.1109/ACCESS.2021.3121543 discloses emotion recognition using a glasses-type wearable device via multi-channel facial responses.

### SUMMARY OF THE INVENTION

The present invention has been made in consideration of the above problem.

According to an aspect of the proposed technique, there is provided a system configured to :
- provide a first signal representing at least movements of the head of a user to a machine learning model and consequently obtain an output representing an emotion of the user, the machine learning model having been trained beforehand using a database of model signals representing movements of a head of a model user and associated with at least an emotion of the model user, and
- process a second signal representing a physiological parameter of the user to confirm the emotion of the user.

The proposed technique allows determining emotions of the user without requiring any conscious interaction from the wearer and using limited hardware. Indeed, the proposed technique does not require a large number of signals representing motion of multiple body markers to be available. Rather, as far as wearer motion is concerned, it is only needed to provide a first signal representing head motion. In some embodiments, the required hardware may even be embedded in a single head-worn device. The specific nature of the first and second signals, combined with the specific approach of first determining an emotion from a machine learning based analysis of the first signal and then confirming the emotion from processing the second signal has surprisingly allowed obtaining better results than in other contemplated approaches.

Optionally, the system further comprises a wearable device comprising sensors configured to sense at least the movements of the head of the user and the physiological parameter of the user when the wearable device is worn by the user. It is more convenient for the wearer when all required sensors are packed in a single head-worn device rather than in multiple devices.

Optionally, the database of model signals represents movements of only the head of the model user. This allows minimizing the storage space required for performing the proposed method.

Optionally, the database of model signals represents movements of the head of the model user more extensively than movements of the body of the model user. This allows providing to a group of wearer an emotion prediction service having different versions depending on the equipment of each wearer. A simpler version of the service is based only on head motion when the wearer is equipped with a single motion sensor embedded in a head-worn device. A more complex version may offer further adjustments accounting for instance on hand motion when the wearer is further equipped with a hand-worn or wrist-worn motion sensor. In order to still achieve good results with the simpler version, it is then recommended that the database of model signals represents movements of the head of the model user extensively enough, especially compared to movements of other body parts of the model user.

Optionally, the system further comprises a control interface configured to generate a control signal based on a condition related to the detected or confirmed emotion.

For instance, a smart eyewear system may be provided to a user, so as to be worn throughout daily activities including for instance walking in a crowd. Such smart eyewear systems may comprise various sensors among which an IMU that may be configured to obtain a first signal as well as camera and sound recording systems that may be configured to obtain pictures, videos, and/or audio recordings. The first signal may be obtained at a current instant and provided in real time to the machine learning model which, in turn, indicates, a detected emotion. A test, such as checking a given criterion, may then be automatically run with the detected emotion. An example of such criterion may be checking if the detected emotion is deemed positive, by corresponding for instance to a positive valence and/or to a high arousal intensity of emotion. In this example, when the detected emotion is deemed positive, the camera may be automatically activated to take a picture, which is labelled according to the detected emotion. Further, during his/her daily activities, the user may further actively take for instance pictures using the camera and may label these pictures depending upon their own perceived emotional state. These labels may be associated to the detected emotion and provided to the machine learning model as labelled training data. Instructions may then be generated to identify the pictures having the most positive emotional ratings among a set of labelled pictures based on their labels. These identified pictures may further be proposed later to the user as an album to print, visualize or share with friends and/or family.

Optionally, the machine learning model may be configured to output, along with a detected emotion, a trust level associated to this detected emotion, and the pictures may be further labelled according to this trust level. When considering the above example, a trust level threshold may be used as a criterion for filtering the labelled pictures. This allows for instance disregarding, any picture having a label representing a detected emotion associated to an insufficient trust level.

Optionally, the system further comprises a wearable device having an activable and/or controllable optical function, for instance a dioptric function and/or a transmission function, the wearable device being configured to activate and/or control the optical function when receiving the control signal.

For instance, a user may be provided with an electro-chromic wearable that is switchable between different possible tints. The choice and comfort of tint appearance may be pre-programmed. Thanks to a user interface application of the electro-chromic wearable, which may be made available for instance on a mobile device, the user may trigger one or another tint management algorithm proposed in relation to his/her perceived needs in terms of light environment management. Separately, a machine learning model may also be run on the user interface application and trained to detect the emotion of the user based on a plurality of signals obtained from sensors on a device worn by the wearer, such sensors including for instance an IMU, a heart rate sensor and a respiratory sensor. The detected emotion may be time-stamped and stored. Further, every tint variation that is manually triggered by the user or automatically triggered by the tint management algorithm may also be identified as an event that is time-stamped and stored. Associating a detected emotion with an event having substantially the same time-stamp, either based on the time-stamps themselves or through other known means, allows improving both the machine learning model that outputs the detected emotion and the tint management algorithm through a co-learning approach. An advantage of this co-learning approach is to avoid facing an inappropriate tint in certain social/environment emotional situations. For example, a user may wish to wear, in principle, a dark tinted eyewear in outside summertime situations. But the user may also wish a clearer tint when in a close social interaction such as a face-to-face discussion with another person, in order to better express his/her emotions with his/her eyes. This allows the other person to improve the level of engagement in the relationship by perceiving and detecting emotion through eye contact. Further, the head dynamics induced by the posture and the motion of such social interaction may help differentiate between different scenarios and allow detecting the emotion. Another advantage of the co-learning approach is that tint variations may be used as user feedback. This includes any tint variation that is manually triggered by the user and any tint variation that is automatically triggered by the tint management algorithm and does not induce a negative reaction by the user such as manually reverting the tint variation or expressing perceivable discomfort through a variation of posture and/or of a physiological parameter.

Optionally, the system further comprises a feedback module, for example a visual, audio and/or haptic feedback module, configured to provide a feedback to the user when receiving the control signal.

Optionally using user feedback, through an interface, to assess a currently detected emotion may help further training the machine learning model. For instance, such feedback may be provided voluntarily by the user by selecting a pre-defined option on an interface and/or as an answer to an automatic notification. The feedback is a user input that may for instance confirm the detected emotion or better represent his/her own self-assessed current emotion. The machine learning model may use this feedback from the user to assess its performance as an asserted level of trust in the detected emotion represented by its output.

Optionally, the system further comprises a messaging module, configured to send a message to the user when receiving the control signal.

Optionally, the system will send a message to the user when detected user emotion will conflict social emotion.

Optionally, the model signals further comprise model contextual data, and the first signal further comprises contextual data.

Optionally, the system further comprises a contextual data module configured to obtain the contextual data, the contextual data module comprising a sensor and/or a communication interface.

Contextual data may include time of day, location, activity data, etc. Optionally, the contextual data comprise an indication of the user wearing a wearable device, and the determined and/or confirmed emotion represents a degree of comfort associated to wearing the wearable device, for example as a rating on a rating scale.

Optionally, the model signals further comprise model user profile data associated to the model user, and the first signal further comprises user profile data associated to the user.

Optionally, the system further comprises a user profile module configured to obtain the user profile data, the user profile module comprising a sensor and/or a communication interface.

Model user profile data and user profile data may include for instance gender, weight, height, age, user identifier, etc.

Optionally, the system further comprises a feeding module configured to feed a user profile with the determined or confirmed emotion, and/or a tagging module configured to tag contextual data based on the determined or confirmed emotion.

For instance, when a detected emotion meets a predetermined criterion, such as being a positively denoted emotion, the camera may be turned on to capture an image of the user. In an example, the camera is embedded in a front side of a spectacle frame worn by the user. Further, the user may be optionally requested to face a mirror before the image is captured. In this example, image processing of the captured image may allow identifying facial expressions and/or a posture of the user, which may be in turn used to confirm the detected emotion. Alternately, a picture of the user may be taken by a friend and shared on a social network. The picture of the user and a detected emotion may be tagged as referring to a same event based for instance on metadata. Crossing the detected emotion with a result of image processing the picture allows confirming the detected emotion.

According to another aspect of the proposed technique, there is provided a method for determining an emotion of a user, the method comprising:
- providing at least a first signal representing movements of the head of the user as an input to a machine learning model and consequently obtaining an output representing an emotion of the user, the machine learning model having been trained beforehand using a database of model signals representing movements of a head of a model user and associated with an emotion of the model user, and
- processing a second signal representing a physiological parameter of the user to confirm the emotion of the user.

According to another aspect of the proposed technique, there is provided a computer program comprising one or more stored sequence/s of instructions that is accessible to a processing unit and which, when executed by the processing unit, causes the processing unit to carry out the above method.

### BRIEF DESCRIPTION OF THE DRAWINGS

For a more complete understanding of the description provided herein and the advantages thereof, reference is now made to the brief descriptions below, taken in connection with the accompanying drawings and detailed description, wherein like reference numerals represent like parts.
FIG. 1 and 2 illustrate schematically machine learning model training algorithms, according to embodiments.
FIG. 3 and 4 illustrate schematically trained machine learning models, according to embodiments.
FIG. 5 illustrate schematically possible uses for a detected and/or a confirmed emotion of a user, according to embodiments.

### DETAILED DESCRIPTION OF THE INVENTION

The proposed technique aims at determining an emotion of the user.

The emotion may be first detected based on a machine learning model being input a signal representing head motion. The output of the machine model may then be confirmed using additional information, which includes a signal representing a physiological parameter of the user.

The proposed technique allows reliably determining the emotion of the user using a minimal combination of sensors. For instance, the signal representing head motion may be obtained from an inertial measurement unit that may be part of a head-worn device, such as eyeglasses, goggles, a headset, an earpiece, and the like, while the signal representing the physiological parameter of the user may be obtained from a physiological sensor that may either be part of the same device or from a different worn device such as a wristband.

The proposed technique may be implemented by running, on a processing unit, a computer program comprising instructions stored on a non-transitory storage medium. When executed by the processing unit, the instructions cause the processing unit to carry out a method according to the proposed technique. The processing unit may further be operably connected to a wide variety of devices through one or more wired or wireless communication interfaces.

For instance, devices operably connected to the processing unit may include one or more sensors of the above combination of sensors. This enables the processing unit to carry out various functions including controlling these sensors and/or processing data sensed by these sensors over time. For instance, devices operably connected to the processing unit may include one or more end devices provided to a user, such end devices comprising for instance one or more user interfaces, for instance a graphical UI, a haptic UI, etc. Such user interfaces enable the processing unit to carry out additional functions including obtaining feedback from a user and/or providing information to a user. More generally, any device operably connected to the processing unit is chosen and provided by the person of ordinary skill in the art depending on which functions are to be achieved by the above computer program.

The proposed technique may find numerous applications. Since both determining an emotion of a user at a given instant and daily life monitoring of such emotions have benefits in various fields. Determining an emotion such as a discomfort of a lens wearer is valuable for decision making orientation in clinical practices (e.g. tele-optometry) or for sales purposes (e.g. digital retail). Determining the emotion of an audience of a media content streamed in a real environment (e.g. smart home) or in a virtual environment (e.g. metaverse), may help determining whether a desired effect is obtained on the audience. In the educational field, learning sequences may be extended or shortened depending on the evolution over time of an assessed level of attention or of fatigue of a learner. Judging of the emotional state of a patient may be used by social robots to adapt their interaction with the patient. For instance, such social robots may help supporting the emotional development of autistic children. Monitoring the emotion of an occupant of a vehicle may be used to trigger specific safety measures, for example through V2X communication, based on preset conditions such as when detecting the driver to be angry.

Definitions are now proposed for a variety of terms and expressions used herein.

The "wearer" designates the user which emotion is to be determined. The wearer wears a head-worn device that comprises, at least, one or more sensors for sensing the first signal.

The "first signal" is an analog or numerical signal which represents translational and/or rotational motion of the head of the wearer. An inertial measurement unit (IMU) is a known combination of sensors which allows measuring motion, by recording timestamped tridimensional positions and orientations, translational and rotational speeds, translational and rotational accelerations. An IMU may be easily integrated in the frame of various mounted devices or wearables and has a low power consumption compared to other types of sensors such as a camera for instance. Possible mounted devices include an IMU clip, smart ears, a true wireless earphone, a smartphone maintained pressed on the head, an AR/VR headset, smart glasses, a smart frame, etc. The first signal may then for instance be a signal output by an IMU embedded in a head-mounted device or wearable.

The "second signal" is an analog or numerical signal which represents a physiological parameter of the user. Well known methods and biomedical sensors allow obtaining a wide range of physiological parameters, including for instance galvanic skin response, skin temperature, analytes from sweat or heart rate variability. Suitable sensors may be arranged in the environment of the user or may be embedded in a variety of worn devices depending on the nature of the physiological parameter at hand and on the context. The second signal may for instance be output by an image processing module receiving as an input an image of the user. In particular the input image may show at least the face of the user. The input image may originate from a camera as an exemplary suitable sensor. The second signal may represent a physiological condition of the user, including for instance flushing, which may be correlated to an emotion or to a list of possible emotions. The second signal may also represent a physiological manifestation of an emotional state or a physiological response to a stimulus. This applies in particular to a variety of facial expressions including smiling. Facial expressions may be extracted by processing a image showing a face of the user and may be correlated to an emotion or to a list of possible emotions. In embodiments, a single head-worn device may embed all required sensors to obtain both the first signal and the second signal.

The first signal and the second signal are not necessarily distinct signals originating from different sensors but may relate to different aspects combined in a single sensed signal. This is the case for instance when an IMU is placed on or close to the skin of the wearer in a position that is appropriate to detect a complex motion that represents the combined effects of head motion, heart rate motion and respiratory motion.

The machine learning model is a model that has been trained to associate one or more inputs, comprising at least the first signal, with a corresponding output variable representing an emotion of the wearer. Training a machine learning model is performed using labelled training data, which in this case are signals that each represent a motion of the head of a model wearer and that are each associated to an emotion of the model wearer. The first signal may be recorded periodically and form a time-series. Based on this time-series, the machine learning model may determine an output variable, representing emotion of the wearer, on various time scales: instantaneous (up to a few seconds), short-term (up to a few minutes) or long term (hours, days, weeks or longer). The machine learning model may further be configured to determine, based on this time-series, a latent variable that represents a durable internal emotional state of the user. This latent variable may be used to determine if the output variable corresponds to a steady, stable emotion state or to a transitory emotional state.

In the field of affective computing, emotion may be classified using appropriate metrics.

A possibility pertaining to supervised training is to define a limited list of categories of possible emotions (for instance angry, fearful, neutral, sad, surprised, disgusted, happy), to label the training data according to these categories, and to train the machine learning model to infer the correct categories when being input with the training data. When labeling the training data, emotion may for instance be classified as a function of very simple markers of head movement as following.
Angry/Fearful ⇒ Head moves up
Neutral/Sad ⇒ No movement / Head moves down
Surprised ⇒ Head moves up
Disgusted ⇒ Head moves down
Happy ⇒ No movement / Head moves up.

A possibility to automatically label the training data without human supervision is to configure a clustering model, for example a neural network, to perform a non-supervised clustering mechanism. Each cluster may then be assigned a corresponding identifier corresponding to a category. The identifier may have a numerical value. The identifier may also be a valence. Valence, also called hedonic tone, designates an exemplary metrics of classification which refers to the intrinsic attractiveness or averseness of an event, object or situation. Valence may be used as a way to characterize emotions so that e.g. anger and fear are negatively valenced while joy is positively valenced. Valence may be assigned a number which possible values are used to label first model signals with as many categories as desired. These values may further be output and associated to the first signal by the machine learning model. The machine learning model may for instance be a binary classifier between a first category regrouping all positively valenced emotions and a second category regrouping all negatively valenced emotions. Classical metrics that may be associated to a binary classifier may include accuracy, sensitivity and specificity. Of course, irrespective of the number of categories of emotions that are considered, the machine learning model may also be a fuzzy classifier or a probabilistic classifier. The machine learning model may address variables and internal emotional states of the wearer according to various possible topologies. As such, the machine learning model may be a linear or a nonlinear Euclidean space classifier.

A technical issue is how to describe the emotion of a wearer from their motion when this motion is detected only from their head. In response to this technical issue, the inventors have determined that head motion, considered as such, can reflect body motion control and execution, as well as emotions induced by social or physical environment interactions. Head motion further provides cues on the wearer that may be decorrelated from their emotion. For instance, analysis of gender-specific differences has been conducted in some motor activities such as walking activities. This analysis has revealed that the dynamic part of full body motion contains information about gender. Since, as already mentioned, head motion reflects such dynamics, knowing the gender of the wearer helps interpreting their head motion and correctly identifying their emotions from their interpreted head motion.

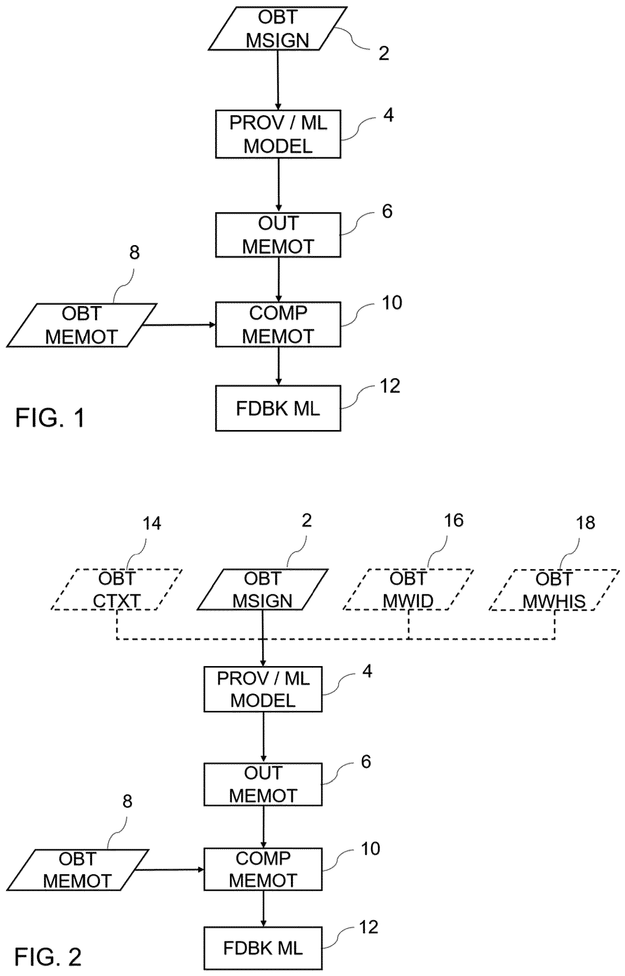

It is now referred to a simple example of a machine learning model training algorithm as illustrated in FIG. 1. Fundamentally, a machine learning model aims at recognizing certain types of patterns. A model is trained over a set of data, thusly providing an algorithm that it can use to reason over and learn from those data. This is the so-called "training phase". Once the model has been trained, it can be used to reason over data that it hasn't seen before, and to make predictions about those data. This is the so-called "production phase". The aim of the training algorithm as illustrated in FIG. 1 is to optimize the model for reconstructing labelled training data. The two main types of machine learning models are supervised and unsupervised. Supervised learning involves learning a function that maps an input to an output based on example input-output pairs. Unsupervised learning, on the contrary, is used to draw inferences and find patterns from input data without references to labeled outcomes. The training algorithm as illustrated in FIG. 1 may indifferently be supervised or unsupervised.

A model signal is obtained (2). The model signal represents at least translational and/or rotational motion of the head of a model wearer, and may further represent motion of other body parts of the model wearer. The model wearer may be an actual wearer, in which case the model signal is sensed. The model wearer may also be representative of an actual wearer or of a group of actual wearers, in which case the model signal is either fully generated or sampled from a database of sensed first signals corresponding to the actual wearer/s. The model signal may be representative of a motor activity of the model wearer, such as a walking activity. The model signal may be acquired upon detecting such an activity and within an appropriate time window that corresponds to the duration of all or part of such an activity. The duration of the time window may depend on contextual data such as the environment of the model wearer or the nature of the activity or may be set to a predefined value that is unrelated to context.

The model signal is further associated, by any known means, to a variable that represents an emotion of the model wearer.

The model signal is provided (4) to the machine learning model which consequently outputs (6) an output variable that represents a prediction of the emotion of the wearer.

The variable associated to the model signal is further obtained (8) and compared (10) to the output variable. The result of the comparison is provided as feedback (12) to the machine learning model. The feedback received after each prediction indicates whether the prediction is correct or incorrect.

The whole process is repeated with different model signals which collectively form the training dataset. Between iterations, the internal logic of the machine learning model is adapted based on the aggregate feedback with the aim of maximizing the number of correct predictions with the training dataset.

It is now referred to a more complex example of a machine learning model training algorithm as illustrated in FIG. 2.

In this more complex example, for each model signal that is provided to the machine learning model for performing a prediction, additional data that is related to this model signal is also provided to the machine learning model. The resulting multimodal and/or temporal analysis which is conducted by the machine learning model generally allows better predictions.

For instance, a set of model signals of a given model wearer may be obtained during an extended period of time during which when the given model wearer performs various activities, such as working, walking, running, sleeping, watching TV, driving, etc. The given model wearer may be further equipped with a device or an interface adapted to sense or more generally obtain, for each model signal, contextual data. Data is said contextual if it refers to an element that may contextualize the first signal and help its interpretation. A contextual element may be an event, a status, a situation, etc. applying to the model wearer and/or to their environment either permanently or temporarily while the model signal was sensed. The contextual data may indicate one or more types of contextual elements, including for instance a relevant physiological parameter of the model wearer, a socio-demographics information of the model wearer such as gender, a facial expression of the model wearer, a noise level in the vicinity of the model wearer, a light level in the vicinity of the model wearer, geolocation data of the model wearer, a timestamp, an activity or task undergone by the model wearer, etc. The contextual data may then be obtained (14) and provided to the machine learning model. The machine learning model may then jointly analyze each model signal for this model wearer with corresponding contextual data at the time of sensing model signal. This allows building correlations. For instance, some head movements may be correlated with specific contextual elements, such as specific activities or groups of similar activities. Emotions may further be correlated with specific contextual elements. Such correlations contribute to the training of the machine learning model and help, specifically, in improving the prediction accuracy of the emotion of the model wearer.

In the training dataset, model signals representing head movement of a given model wearer may further be tagged with an identifier of said given model wearer. Further obtaining (16) and providing these identifiers to the machine learning model allows the model to build correlations that are specific to a model wearer or to a group of model wearers between their head motion patterns and their emotions. As a consequence, after the training phase, the machine learning model may analyze a first signal differently, and consequently output a different output variable, depending on whether this first signal represents head motion of a first wearer or of a second wearer.

Model signals representing head motion of the same model wearer may further be organized and provided to the machine learning model as a set of time series which relate to different aspects of the head motion. In other words, when considering a model signal corresponding to a given point in time that is obtained (2) and provided (4) to the machine learning model, it is possible to further obtain (18) and provide to the machine learning model one or more historical model signals corresponding to one or more earlier point in time. This allows the machine learning model to interpret not simply an instantaneous indication but rather a sequence of successive indications of the head motion of the model wearer up to the given point in time. The machine learning model may then reliably identify various types of data from temporal, and possibly multimodal, correlations between successive model signals relating to successive points in time up to the given point in time. In turn, the machine learning model may then associate the thusly identified types of data with a corresponding emotion and output the output variable accordingly. An example of type of data that may be identified is for instance a head motion pattern of the model wearer, such as moving head up for a brief moment then moving head back down again. Another example of type of data that may be identified is an estimation or confirmation of a contextual element, such as a detected vibration that is characteristic of the model wearer riding a bicycle. Yet another example of type of data that may be identified is an estimation of overall dynamic body acceleration as a measure reflecting energy consumption of the model wearer. Indeed, as already mentioned analyzing head motion may provide indications on body motion as a whole.

A proof-of-concept for extrapolating emotions based on head motion only has been developed by the inventors and is now described. Code illustrating movements of 15 body markers of model walkers, as provided by BiomotionLab on their website https://www.biomotionlab.ca/html5-bml-walker/, has been retrieved. From the retrieved code, for each of the 15 body markers, corresponding motion data has been regenerated for different full scaled emotions respectively labelled "sad", "happy" and "neutral". A database has then been built associating the motion data with the labelled emotion, and the database has been split in a training dataset and a test dataset. A logistic regression model has been trained using the training database and the trained model has been applied on the test dataset. Recognition scores expressed as AUC values (AUC standing for "area under the curve") have been determined. On the "happy" and "sad" emotions, the recognition score is 0.65 with head data only and 0.67 with head + hands. A higher recognition score of 0.73 has been achieved for the neutral emotion.

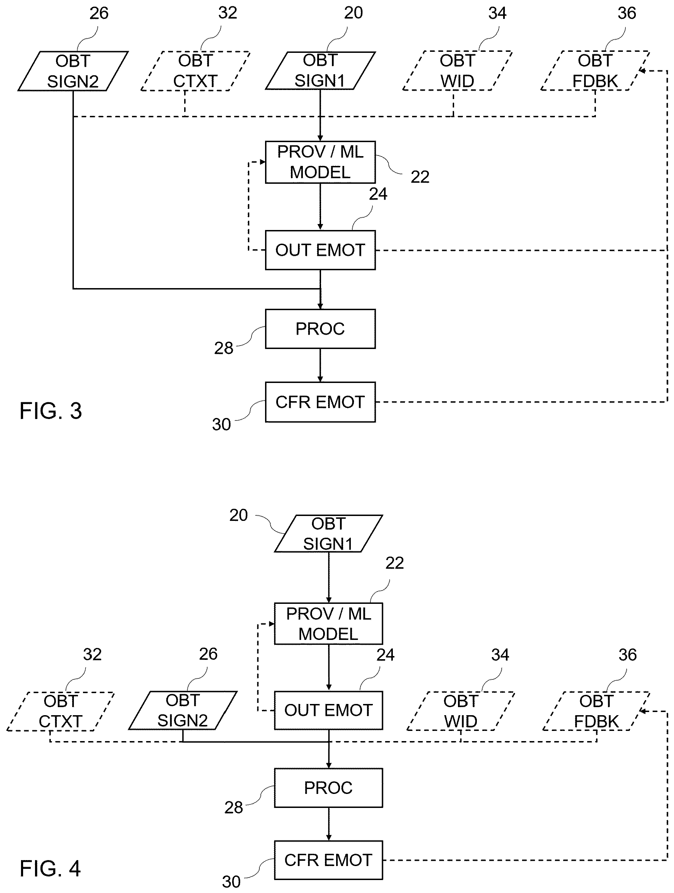

It is now referred to FIG. 3 and FIG. 4, which describe examples of possible uses in a production phase of the machine learning model having been previously trained as depicted for instance in FIG. 1 or FIG. 2.

The first signal is first obtained (20) and provided (22) to the machine learning model. The machine learning model may for instance extract one or more of the following data from the first signal: head acceleration, speed, frequency-amplitude analysis, entropy, jerk and analyze the extracted data to identify a pattern that is also found in the analysis of the training data, and to consequently output (24) the output variable.

Various additional data can be further obtained and provided to the machine learning model in order to benefit from the correlations identified in the training phase and better distinguish between similar emotions. Reliability enhancement and disambiguation may result for example from data fusion from multiple sensors.

Obtaining (26) and providing the second signal to the model may allow the model to extract relevant data from the second signal, identify a pattern in the multimodal data extracted from the first signal and from the second signal, compare the identified pattern with patterns that have also been found in the analysis of the training data, and consequently output (24) the output variable. The same principle of a multimodal analysis for enhanced results applies when obtaining (32) and providing contextual data to the model or when obtaining (34) and providing a wearer identifier to the model.

The wearer identifier may be combined with user or wearer profile elements and may be processed by the machine learning model to customize the emotion determination. For instance, kinematics and dynamics of head motion may vary between individuals irrespective of their emotions. For instance, let us consider that a downward head movement may be associated to a negative emotion such as disgust or sadness. When the machine learning model is neither provided with any wearer identifier nor with any wearer profile element associated to the first signal, it is likely that the machine learning model simply sets one or more thresholds, for instance in terms of amplitude of a downward angle or angular speed which, when exceeded, leads to outputting an output variable representing such negative emotion. When, on the contrary, the machine learning model is provided with a wearer identifier or with a wearer profile element associated to the first signal, a further possibility is that different values of the above threshold/s are set for different wearers.

Assuming a walking activity for instance, an estimation of stance swing and of walking speed, as derived from additional data, may be combined with the first signal and allow the machine learning model to decorrelate a part of head dynamics, contained in the first signal, that is solely or mostly due to the walking activity and another part of head dynamics, also contained in the first signal, that is solely or mostly due to emotion of the wearer.

Additional data provided for instance by sensors may further help identifying co-events, such as climbing stairs or mixing with a fast-moving crowd, that may contribute to detecting an emotional transition of the wearer.

Additional data relating to context such as the environment or the activity of the wearer, to daily time, to previous values of the output variable, etc. may further be used to pre-process or filter an incoming succession of first signals. For instance, it is possible to set one or more criteria that may be met or not by the additional data and to trigger different actions depending on whether the criteria are met or not. A possible action is to provide the first signal to the machine learning model only when one or more criteria are met and, conversely, not to provide the first signal to the machine learning model when one or more criteria are not met. Another possible action is to modify, for example tag, the first signal before providing it the machine learning model depending on whether a certain criterion is not met. Considering the example of a criterion based on daily time, it is possible to preprocess all first signals by tagging them with tags representing daytime or nighttime before providing the tagged first signals to the machine learning model.

The output variable may be stored for further use by the machine learning model. In particular, emotion kinematic patterns may have been identified in the training data, so that the output variable representing the emotion of the wearer at a given point in time may also be a hint for:
- determining the emotion of the wearer in the near future, or
- determining whether the emotion of the wearer is stable or not across a set amount of time, or
- for detecting an event that causes for the wearer an emotional transition that is either short-term or long-term, in other words which relates to the output variable that represents an apparent, or current, emotion of the user or to the latent variable that represents a durable emotion of the user.

The output variable and/or the latent variable may then be associated to a variety of parameters among which an indication of whether the represented emotion is steady/stable or transitional. When the represented emotion is transitional, an indication of the kinematics of the transition may be further provided. When the represented emotion is stable, an indication of a duration and/or of an initial and a final time associated to the represented emotion may be further provided. Such indications allow an enhanced downstream management of the emotion of the wearer.

Another example of further use of the stored output variable is in combination with requesting feedback from the wearer on the emotion represented by the stored output variable. The request may be a popup window or an audio or haptic signal, etc. prompting the wearer to interact with a human-machine interface. The feedback may then be obtained (36) in the form of a signal resulting of such an interaction or of an absence of such an interaction before expiration of a set timer. The feedback may confirm or dispute the emotion represented by the stored output variable. The feedback may be associated to the stored output variable and provided to the machine learning model in order to contribute to further training the model. In particular, providing wearer feedback associated to a wearer identifier allows the machine training model to continue learning from individual data.

In addition to the output variable being output (24) by the machine learning model, additional data is further obtained (26, 32, 34, 36) and processed (28) to confirm (30) the emotion of the wearer. The additional data includes at least the second signal and may further include contextual data, wearer identifier or feedback on the confirmed emotion.

A possible way of processing (28) the second signal is now described by means of example. For instance, the second signal may be output by a pupillometer. A lookup table may be provided with a list of entries, each entry mapping a list of allowable values of the output variable with a corresponding range of pupil size values. In this example, processing the second signal may comprise identifying, from the second signal, a range of pupil size which the pupil size of the wearer belongs to, then retrieving in the lookup table the entry corresponding to the identified range. Next, it may be checked whether the value of the output variable is found in the list of allowable values. The emotion of the wearer may then be confirmed if the result of the check is positive or disputed if the result of the check is negative. Of course, the same principle may be applied to process a second signal input by any other sensor or to process any other type of additional data. For instance, if second signals are received from sensors such as a heart rate monitor and/or a breath rate sensor, then the entries of the lookup table may relate to ranges of heart rate values and/or of breath rate values.

Alternately, the machine learning model may be conceptually separated in a first internal model receiving at least the first signal and outputting (24) the output variable and a second internal model receiving and processing (28) the second signal to output a processing result. The output variable and the output processing result may then be processed together to confirm (30) the emotion represented by the output variable.

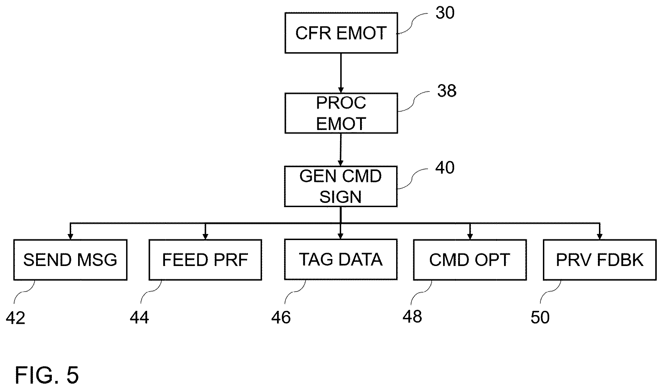

It is now referred to FIG. 5 which provides a non-exhaustive list of possible uses for the detected and confirmed emotion of the user.

After the emotion is confirmed (30), the output variable may be processed (38) to generate (40) a control signal that may contain instructions for a variety of hardware and/or software modules.

A messaging service receiving the control signal may then send (42) appropriate messages at appropriate moments, for instance via an application and/or a device, for instance via a worn device such as a mixed reality headset. The messages may be meant for the wearer or for other users. The messages may convey positive reinforcement of positive emotions that have been detected and confirmed, and may also convey alerts, warnings or suggestions when negative emotions are detected and confirmed.

A wearer visual profile manager may create and feed (44) a visual live, ergonomic, wearer experience profile comprising a set of preferences that are automatically adjusted for the wearer in real-time based on the output variable. The adjustment may be further based for instance on contextual data including ambient light level. The adjustment may be further based on the latent variable. The visual profile may be automatically applied when operating a device having a visual interface, such as a computer, a tablet, a smartphone, an active optical element placed before an eye or both eyes of the wearer, a head-mounted device adapted to display visual elements to the wearer, etc.

A data labeller may be provided with timestamped data. The control signal may comprise instructions for the data labeller to label (46) any timestamped data having a timestamp corresponding to a time associated to an output variable representing a detected emotional event for the wearer. Several types of timestamped data may be accessed and labelled by the data labeller, including timestamped data related to a situation, such as collected pictures, environmental features, visual ergonomics specifications, visual, emotional and motor tasks, timestamped physical data related to user, such as geolocation, head motion, lighting, weather , crowding, timestamped dataflows such as music, phone calls, messages, application notifications, etc.

An active optical function controller may control a variety of optical functions, including activating filters, displaying images in augmented and/or virtual reality, activating or adjusting a transmission function to achieve for instance a desired tint and/or color intensity, controlling a dioptric function to achieve for instance a magnification or a focus in a specific gazing direction and/or for a specific gazing distance, etc. The control signal may comprise instructions for the active optical function controller to control (48) the optical function in a differentiated manner depending at least on the output variable, and optionally further based on the latent variable, contextual data, wearer profile data, etc. As a result, it is possible to bridge for instance lifestyle, behavior and emotion of a wearer and to customize accordingly an optical function for the wearer. It is further possible to detect and confirm an emotion as well as a simultaneous appearance of a specific element in the field of view of the wearer, and as a consequence control an active dioptric function in a specific gazing direction and/or at a specific gazing distance forming a region of interest corresponding to the location of the specific element in the field of view of the wearer. It is further possible to detect and confirm an emotion and obtain simultaneous contextual data relating to the environment of the wearer, such as an abrupt change in ambient light intensity, and as a consequence trigger a change in a transmission function to compensate this abrupt change.

A feedback module may be configured to provide (50) feedback to a user on their environment, contextual data, tasks, long-term emotion, etc. A possible feedback example may correspond to automatically taking a picture using a camera embedded on a head-worn device or recording a sound using an embedded microphone whenever the output variable represents for instance happiness of the wearer and the latent variable further represents emotional invariance over a sufficient amount of time. The taken picture and/or the recorded sound may then be stored to be later retrieved and provided to the user.

All the above modules may be activated and modulated in a differentiated manner for each possible emotion of the wearer that may be represented by the output variable. Further differentiation may be based on whether the represented emotion is stable or transitory.

A few use cases are now briefly described.

In a first use case, a wearer is deemed focused on a task and a zonal transmission function is activated by default to dim peripheral light in order to avoid disturbing the wearer and to help the wearer focus on the task. Throughout the task, first signals are obtained periodically, for instance every few seconds or every minute. The first signals may be filtered to conform to specific measurement conditions, that is to say that if the specific measurement conditions are met for a given first signal, then the given first signal is provided to the machine learning model, otherwise the given first signal is disregarded. An exemplary purpose of such filtering as a pre-processing act may be to eliminate outliers. For each provided first signal, the machine learning model outputs a corresponding output variable that represents the emotion of the wearer as derived at least from the analysis of the provided first signal. The output variables are collectively interpreted to monitor the emotional state of the wearer over time and more specifically to check wellness or boredom of the wearer. When boredom is detected, a possible action may be to automatically suggest to the wearer to pause the task by an appropriate message. Another possible action may be to interrupt the dimming of peripheral light in order to further encourage the wearer to have a break.

In a second use case, a consumer tries on eyeglasses physically or virtually. The consumer may verbally express a subjective emotion while trying the spectacle frame. An objective emotion may further be detected by the machine learning model being provided a first signal and outputting an output variable. The objective emotion may further be confirmed by processing the second signal. The confirmed objective emotion and the expressed subjective emotion may be compared to identify a concordance or a discordance. In case of a discordance, for example when the subjective emotion is positive but the objective emotion is not, it is possible to generate an alert or a recommendation for trying on another spectacle frame despite the positive subjective emotion.

## Claims

1. A system configured to :
- provide (22) a first signal representing at least movements of the head of a user to a machine learning model and consequently obtain (24) an output representing a detected emotion of the user, the machine learning model having been trained beforehand using a database of model signals representing movements of a head of at least one model user and associated with at least an emotion of the at least one model user, and
- process (28) a second signal representing a physiological parameter of the user to obtain (30) a confirmed emotion of the user.

2. The system according to claim 1, wherein the system further comprises a wearable device comprising sensors configured to sense (20, 26) at least the movements of the head of the user and the physiological parameter of the user when the wearable device is worn by the user.

3. The system according to claim 1 or 2, wherein:
- the database of model signals represents movements of only the head of the model user, or
- the database of model signals represents movements of the head of the model user more extensively than movements of the body of the model user.

4. The system according to any one of claims 1 to 3, wherein the system further comprises a control interface configured to generate (40) a control signal based on the detected emotion of the user or on the confirmed emotion of the user.

5. The system according to claim 4, wherein the system further comprises a wearable device having an activable and/or controllable optical function, the wearable device being configured to activate and/or control (48) the optical function according to the control signal.

6. The system according to claim 4 or 5, wherein the system further comprises a feedback module configured to receive (36) a feedback from the user of the detected emotion of the user or of the confirmed emotion of the user.

7. The system according to any one of claims 4 to 6, wherein the system further comprises a messaging module, configured to send (42) a message to the user according to the control signal.

8. The system according to any one of claims 1 to 7, wherein the model signals further comprise model contextual data, and the first signal further comprises contextual data.

9. The system according to claim 8, wherein:
- the contextual data comprise an indication of the user wearing a wearable device, and
- the determined emotion of the user and/or the confirmed emotion of the user represents a degree of comfort associated to wearing the wearable device.

10. The system according to claim 8 or 9, wherein the system further comprises a contextual data module configured to obtain (32) the contextual data, the contextual data module comprising a sensor and/or a communication interface.

11. The system according to any one of claims 1 to 10, wherein the model signals further comprise model user profile data associated to the model user, and the first signal further comprises user profile data associated to the user.

12. The system according to claim 11, wherein the system further comprises a user profile module configured to obtain (34) the user profile data, the user profile module comprising a sensor and/or a communication interface.

13. The system according to any one of claims 1 to 12, the system further comprising:
- a feeding module configured to feed (44) a user profile with the determined or confirmed emotion, and/or
- a tagging module configured to tag (46) contextual data based on the determined or confirmed emotion.

14. A method for determining an emotion of a user, the method comprising:
- providing (22) at least a first signal representing movements of the head of the user as an input to a machine learning model and consequently obtaining (24) an output representing a detected emotion of the user, the machine learning model having been trained beforehand using a database of model signals representing movements of a head of at least one model user and associated with an emotion of the at least one model user, and
- processing (28) a second signal representing a physiological parameter of the user to obtain (30) a confirmed emotion of the user.

15. A computer program comprising one or more stored sequence/s of instructions that is accessible to a processing unit and which, when executed by the processing unit, causes the processing unit to carry out a method according to claim 14.

## Patentansprüche

1. System, ausgelegt zu Folgendem:
- Bereitstellen (22) eines ersten Signals, das mindestens Bewegungen des Kopfes eines Benutzers darstellt, an ein Maschinenlernmodell und folglich Erhalten (24) einer Ausgabe, die eine erkannte Emotion des Benutzers darstellt, wobei das Maschinenlernmodell zuvor unter Verwendung einer Datenbank von Modellsignalen trainiert wurde, die Bewegungen eines Kopfes mindestens eines Modellbenutzers darstellen und mit mindestens einer Emotion des mindestens einen Modellbenutzers assoziiert sind, und
- Verarbeiten (28) eines zweiten Signals, das einen physiologischen Parameter des Benutzers darstellt, um eine bestätigte Emotion des Benutzers zu erhalten (30).

2. System nach Anspruch 1, wobei das System ferner eine tragbare Vorrichtung umfasst, die Sensoren umfasst, die dazu ausgelegt sind, mindestens die Bewegungen des Kopfes des Benutzers und den physiologischen Parameter des Benutzers zu erfassen (20, 26), wenn die tragbare Vorrichtung von dem Benutzer getragen wird.

3. Verfahren nach Anspruch 1 oder 2, wobei:
- die Datenbank von Modellsignalen nur Bewegungen des Kopfes des Modellbenutzers darstellt, oder
- die Datenbank von Modellsignalen Bewegungen des Kopfes des Modellbenutzers umfassender darstellt als Bewegungen des Körpers des Modellbenutzers.

4. System nach einem der Ansprüche 1 bis 3, wobei das System ferner eine Steuerschnittstelle umfasst, die dazu ausgelegt ist, ein Steuersignal basierend auf der erkannten Emotion des Benutzers oder auf der bestätigten Emotion des Benutzers zu erzeugen (40).

5. System nach Anspruch 4, wobei das System ferner eine tragbare Vorrichtung mit einer aktivierbaren und/oder steuerbaren optischen Funktion umfasst, wobei die tragbare Vorrichtung dazu ausgelegt ist, die optische Funktion gemäß dem Steuersignal zu aktivieren und/oder zu steuern (48).

6. System nach Anspruch 4 oder 5, wobei das System ferner ein Rückmeldungsmodul umfasst, das dazu ausgelegt ist, von dem Benutzer eine Rückmeldung der erkannten Emotion des Benutzers oder der bestätigten Emotion des Benutzers zu empfangen (36).

7. System nach einem der Ansprüche 4 bis 6, wobei das System ferner ein Nachrichtenübermittlungsmodul umfasst, das dazu ausgelegt ist, eine Nachricht gemäß dem Steuersignal an den Benutzer zu senden (42).

8. System nach einem der Ansprüche 1 bis 7, wobei die Modellsignale ferner Modellkontextdaten umfassen und das erste Signal ferner Kontextdaten umfasst.

9. System nach Anspruch 8, wobei:
- die Kontextdaten eine Angabe umfassen, dass der Benutzer eine tragbare Vorrichtung trägt, und
- die bestimmte Emotion des Benutzers und/oder die bestätigte Emotion des Benutzers einen Grad an Komfort darstellt, der mit dem Tragen der tragbaren Vorrichtung verbunden ist.

10. System nach Anspruch 8 oder 9, wobei das System ferner ein Kontextdatenmodul umfasst, das dazu ausgelegt ist, die Kontextdaten zu erhalten (32), wobei das Kontextdatenmodul einen Sensor und/oder eine Kommunikationsschnittstelle umfasst.

11. System nach einem der Ansprüche 1 bis 10, wobei die Modellsignale ferner Modellbenutzerprofildaten umfassen, die mit dem Modellbenutzer assoziiert sind, und das erste Signal ferner Benutzerprofildaten umfasst, die mit dem Benutzer assoziiert sind.

12. System nach Anspruch 11, wobei das System ferner ein Benutzerprofilmodul umfasst, das dazu ausgelegt ist, die Benutzerprofildaten zu erhalten (34), wobei das Benutzerprofilmodul einen Sensor und/oder eine Kommunikationsschnittstelle umfasst.

13. System nach einem der Ansprüche 1 bis 12, wobei das System ferner Folgendes umfasst:
- ein Einspeisemodul, das dazu ausgelegt ist, die bestimmte oder bestätigte Emotion in ein Benutzerprofil einzuspeisen (44), und/oder
- ein Kennzeichnungsmodul, das dazu ausgelegt ist, kontextbezogene Daten basierend auf der bestimmten oder bestätigten Emotion zu kennzeichnen (46).

14. Verfahren zum Bestimmen einer Emotion eines Benutzers, wobei das Verfahren Folgendes umfasst:
- Bereitstellen (22) mindestens eines ersten Signals, das Bewegungen des Kopfes des Benutzers darstellt, als eine Eingabe in ein Maschinenlernmodell und folglich Erhalten (24) einer Ausgabe, die eine erkannte Emotion des Benutzers darstellt, wobei das Maschinenlernmodell zuvor unter Verwendung einer Datenbank von Modellsignalen trainiert wurde, die Bewegungen eines Kopfes mindestens eines Modellbenutzers darstellen und mit einer Emotion des mindestens einen Modellbenutzers assoziiert sind, und
- Verarbeiten (28) eines zweiten Signals, das einen physiologischen Parameter des Benutzers darstellt, um eine bestätigte Emotion des Benutzers zu erhalten (30).

15. Computerprogramm, umfassend eine oder mehrere gespeicherte Sequenz/en von Anweisungen, auf die durch eine Verarbeitungseinheit zugegriffen werden kann und die, wenn sie von der Verarbeitungseinheit ausgeführt werden, die Verarbeitungseinheit zum Durchführen eines Verfahrens nach Anspruch 14 veranlassen.

## Revendications

1. Système configuré pour :
- fournir (22) un premier signal représentant au moins les mouvements de la tête d'un utilisateur à un modèle d'apprentissage automatique et obtenir (24) en conséquence une sortie représentant une émotion détectée de l'utilisateur, le modèle d'apprentissage automatique ayant été préalablement entraîné à l'aide d'une base de données de signaux modèle représentant les mouvements de la tête d'au moins un utilisateur modèle et associés à au moins une émotion de l'au moins un utilisateur modèle, et
- traiter (28) un deuxième signal représentant un paramètre physiologique de l'utilisateur pour obtenir (30) une émotion confirmée de l'utilisateur.

2. Système selon la revendication 1, dans lequel le système comprend en outre un dispositif portable comprenant des capteurs configurés pour détecter (20, 26) au moins les mouvements de la tête de l'utilisateur et le paramètre physiologique de l'utilisateur lorsque le dispositif portable est porté par l'utilisateur.

3. Procédé selon l'une des revendications 1 ou la revendication 2, dans lequel :
- la base de données de signaux modèle représente les mouvements de la tête de l'utilisateur modèle uniquement, ou
- la base de données de signaux modèle représente les mouvements de la tête de l'utilisateur modèle de manière plus étendue que les mouvements du corps de l'utilisateur modèle.

4. Système selon l'une quelconque des revendications 1 à 3, dans lequel le système comprend en outre une interface de commande configurée pour générer (40) un signal de commande basé sur l'émotion détectée de l'utilisateur ou sur l'émotion confirmée de l'utilisateur.

5. Système selon la revendication 4, dans lequel le système comprend en outre un dispositif portable ayant une fonction optique activable et/ou contrôlable, le dispositif portable étant configuré pour activer et/ou contrôler (48) la fonction optique en fonction du signal de commande.

6. Système selon la revendication 4 ou la revendication 5, dans lequel le système comprend en outre un module de retour configuré pour recevoir (36) un retour de l'utilisateur de l'émotion détectée de l'utilisateur ou de l'émotion confirmée de l'utilisateur.

7. Système selon l'une quelconque des revendications 4 à 6, dans lequel le système comprend en outre un module de messagerie, configuré pour envoyer (42) un message à l'utilisateur en fonction du signal de commande.

8. Système selon l'une quelconque des revendications 1 à 7, dans lequel les signaux modèle comprennent en outre des données contextuelles modèle, et le premier signal comprend en outre des données contextuelles.

9. Système selon la revendication 8, dans lequel :
- les données contextuelles comprennent une indication que l'utilisateur porte un dispositif portable, et
- l'émotion déterminée de l'utilisateur et/ou l'émotion confirmée de l'utilisateur représentent un degré de confort associé au port du dispositif portable.

10. Système selon la revendication 8 ou la revendication 9, dans lequel le système comprend en outre un module de données contextuelles configuré pour obtenir (32) les données contextuelles, le module de données contextuelles comprenant un capteur et/ou une interface de communication.

11. Système selon l'une quelconque des revendications 1 à 10, dans lequel les signaux modèle comprennent en outre des données de profil utilisateur modèle associées à l'utilisateur modèle, et le premier signal comprend en outre des données de profil utilisateur associées à l'utilisateur.

12. Système selon la revendication 11, dans lequel le système comprend en outre un module de profil utilisateur configuré pour obtenir (34) les données de profil utilisateur, le module de profil utilisateur comprenant un capteur et/ou une interface de communication.

13. Système selon l'une quelconque des revendications 1 à 12, le système comprenant en outre :
- un module d'alimentation configuré pour alimenter (44) un profil utilisateur avec l'émotion déterminée ou confirmée, et/ou
- un module de marquage configuré pour marquer (46) des données contextuelles en fonction de l'émotion déterminée ou confirmée.

14. Procédé pour déterminer une émotion d'un utilisateur, le procédé comprenant les étapes suivantes :
- fournir (22) au moins un premier signal représentant les mouvements de la tête de l'utilisateur en tant qu'entrée à un modèle d'apprentissage automatique et obtenir (24), en conséquence, une sortie représentant une émotion détectée de l'utilisateur, le modèle d'apprentissage automatique ayant été préalablement entraîné à l'aide d'une base de données de signaux modèle représentant les mouvements de la tête d'au moins un utilisateur modèle et associés à une émotion de l'au moins un utilisateur modèle, et
- traiter (28) un deuxième signal représentant un paramètre physiologique de l'utilisateur pour obtenir (30) une émotion confirmée de l'utilisateur.

15. Programme informatique comprenant une ou plusieurs séquences stockées d'instructions qui sont accessibles à une unité de traitement et qui, lors de son exécution par l'unité de traitement, amène l'unité de traitement à exécuter un procédé selon la revendication 14.
